# EUROPEAN PATENT SPECIFICATION

(11) **EP 3 634 389 B1**
(45) Date of publication and mention of the grant of the patent: **01.05.2024**
(21) Application number: 18726951.9
(22) Date of filing: 16.05.2018
(51) Int. Cl.: A61K 9/68, A61K 9/28, A61K 31/465, A61P 25/26

(54) **NICOTINE-CONTAINING CHEWING GUM COMPOSITIONS**
NIKOTINHALTIGE KAUGUMMIZUSAMMENSETZUNGEN
COMPOSITIONS DE GOMME À MÂCHER CONTENANT DE LA NICOTINE

(30) Priority: 30.05.2017 SE 1750679
(43) Date of publication of application: 15.04.2020
(60) Divisional application: 22176776.7
(73) Proprietor: Enorama Pharma AB, 211 34 Malmö (SE)
(72) Inventor: RUDRARAJU, Varma, Hyderabad 500 014 (IN); RAJESH, Yelchuri, Hyderabad 500 014 (IN)
(74) Representative: AWA Sweden AB
(86) International application number: PCT/EP2018/062749
(87) International publication number: WO 2018/219650

(56) References cited:
- WO-A1-2009/007770
- WO-A1-2010/121619
- WO-A1-2013/056709
- WO-A2-2009/141321
- WO-A2-2009/143845

## Description

### FIELD OF THE INVENTION

The application relates to a chewing gum product for delivering nicotine to a subject. The present application also provides a system for delivering nicotine as well as a method for production of said chewing gum product.

### BACKGROUND OF THE INVENTION

In recent years, with the recognition of the harmful effects of tobacco smoking, there have been numerous campaigns and programs by governmental agencies and various health groups and other interested organizations to disseminate information about the adverse health effects resulting from tobacco smoking. Moreover, and as a result of this recognition of the harmful effects, there have been many programs directed to attempts in reducing smoking incidence.

Nicotine is an organic compound and is the principal alkaloid of tobacco. Nicotine is the chief addictive ingredient in the tobacco used in cigarettes, cigars, snuff and the like. Nicotine is also an addictive drug, though, and smokers characteristically display a strong tendency to relapse after having successfully stopped smoking for a time. Nicotine is the world's second most used drug, after caffeine from coffee and tea.

The main problem with tobacco smoking is its enormous implications on health. Today it is estimated that smoking related diseases cause some 3-4 million deaths per year. In fact, excessive smoking is now recognized as one of the major health problems throughout the world. This grim consequence of tobacco smoking has urged many medical associations and health authorities to take very strong actions against the use of tobacco.

One way to reduce smoking is to provide nicotine in a form or manner other than by smoking and some products have been developed to fulfill this need. Nicotine containing formulations are currently the dominating treatments for tobacco dependence.

As an aid for those who are willing to stop smoking there are several ways and forms of nicotine replacement products available on the market. Several methods and means have been described for diminishing the desire of a subject to use tobacco, which comprises the step of administering to the subject nicotine or a derivative thereof as described in e.g., U.S. Pat. No. 5,810,018 (oral nicotine spray), U.S. Pat. No. 5,939,100 (nicotine containing microspheres) and U.S. Pat. No. 4,967,773 (nicotine containing lozenge) and nicotine containing chewing gums.

Nicotine-containing nose drops have been reported (Russell et al., British Medical Journal, Vol. 286, p. 683 (1983); Jarvis et al., Brit. J. of Addiction, Vol. 82, p. 983 (1987)). Nose drops, however, are difficult to administer and are not convenient for use at work or in other public situations. Ways of administrating nicotine by way of delivering directly into the nasal cavity by spraying is known from U.S. Pat. No. 4,579,858, German Patent No. 32 41 437 and International Publication No. WO/9312764. There may, though, be local nasal irritation with use of nasal nicotine formulations. The difficulty in administration also results in unpredictability of the dose of nicotine administered.

The use of skin patches for transdermal administration of nicotine has been reported (Rose, in Pharmacologic Treatment of Tobacco Dependence, (1986) pp. 158-166, Harvard Univ. Press). Nicotine-containing skin patches that are in wide use today can cause local irritation and the absorption of nicotine is slow and affected by cutaneous blood flow.

Also, inhaling devices resembling a cigarette are known for uptake of nicotine vapours as suggested in U.S. Pat. No. 5,167,242. Said means and methods address the problems associated with addiction to nicotine.

One successful product that is used as a smoking substitute and/or as a smoking cessation aid and which is based on nicotine is the chewing gum Nicorette^{™}. This product was one of the first nicotine replacement forms that was approved by the Food and Drug Administration (FDA) and is still one of the most used nicotine replacement products. Nicorette^{™} chewing gum has been on the market in about 60 countries for several years. In this chewing gum the nicotine is present in the form of a complex with an insoluble cation-exchanger (polacrilex) that is dispersed in a gum base. The nicotine is slowly released from the gum due to chewing and will reach similar plasma levels as when smoking a cigarette after about 30 minutes depending on the chewing technique, i.e., slow or active. Patents related to this product are, e.g., the U.S. Pat. Nos. 3,877,468, 3,901,248 and 3,845,217.

WO 98/23165 discloses a chewing gum wherein nicotine may be in the coating. This concept may provide rapid release of the nicotine from the coated chewing gum, but not a sufficiently rapid buccal uptake of the nicotine. The fraction of the released nicotine that is not immediately absorbed will be flushed down in the gastrointestinal (G.I.) tracts by the saliva, thereby possibly causing hiccups and other G.I. side effects. Once absorbed by the G.I. route this swallowed nicotine will be subjected to first pass metabolism.

WO 00/13662 discloses a chewing gum for systemic, oral administration of an active whereby said active is administered by the chewing gum composition in a bi-phasic manner. The bi-phasic delivery is obtained by the gum matrix as such, not from any coating. WO 00/19977 discloses a substantially moisture free and possibly coated chewing gum for delivery of an active. The possible coating is not said to be buffered. In WO 02/102357, assigned to Pharmacia AB, discloses a nicotine-containing chewing gum product comprising at least one coating, where the coating is buffered.

In WO 00/35298 discloses methods for producing a chewing gum that is said to release an active agent in a controlled way, by physically modifying the release properties of the active agent by coating and drying.

In US 4,828,845, assigned to Warner-Lambert, discloses a process for producing a coated chewing gum, whereby at least three coating solutions are applied one after the other by spraying on to a chewing gum core.

In WO2006124366, by Warner-Lambert, discloses a chewing gum which results in a long lasting effect of flavouring agent, dominant to the effect of flavouring agent in core but higher control of the release of the drug and of non-active excipients by employing polymers like HPMC, HPC in the coating. WO 2009/141321 A2 discloses chewing gum composition for use in nicotine replacement therapy which provides a rapid nicotine release with a high rate of buccal absorption.

However, typical nicotine chewing gums known so far have turned out to be dosage forms which release nicotine rather slowly and thus are not ideal to promote rapid transmucosal absorption. Furthermore, even when providing nicotine chewing gums with rapid release of nicotine, it is found that the rapid release of nicotine did not translate into fast buccal absorption of nicotine.

Hence, there is a need in the art for an improved delivery system for nicotine from chewing gum to suppress the nicotine cravings of consumer adequately in the areas of improved nicotine release and adsorption through oral mucosa simultaneously mimicking the taste of the nicotine.

### SUMMARY OF THE INVENTION

Thus, the object of the present invention is to provide improved and stable nicotine-containing chewing gum compositions that will overcome the drawbacks in the prior art and will sufficiently rapidly satisfy the craving that most smokers experience.

The present invention provides a stable chewing gum for use in nicotine replacement therapy comprising:i) a core having a) a granular region comprising a compressible chewing gum base that is suitable for tabletting at ambient temperature, at least one nicotine active, and optionally, one or more pharmaceutically acceptable ingredients; and b) an extragranular region comprising buffering agents and optionally, one or more pharmaceutically acceptable ingredients; ii) an inner coating layer comprising at least one polyol; and iii) an outer flavored coating present on top of the polyol coat and surrounding the core.

The present invention provides nicotine chewing gums that can be manufactured by tabletting techniques, and thus, do not require the specific (e.g. melt extrusion) equipment necessary in the manufacture of conventional chewing gums.

Further, in another embodiment, the present invention provides efficient and effective chewing gum compositions for use in nicotine replacement therapy, which provide rapid nicotine release in the oral cavity and fast--but non-toxic, pharmaceutically acceptable buccal absorption of nicotine, and which is characterized by comprising a compressible chewing gum base that allows the chewing gum to be manufactured by tabletting at least one nicotine active and at least one buffering agent.

### DETAILED DESCRIPTION OF THE INVENTION

The present invention relates to stable nicotine-containing chewing gum compositions and the method of preparing the same.

The present invention provides chewing gum for use in nicotine replacement therapy comprising:i) a core having a) a granular region comprising a compressible chewing gum base that is suitable for tabletting at ambient temperature, at least one nicotine active, and optionally, one or more pharmaceutically acceptable ingredients; and b) an extragranular region comprising buffering agents and optionally, one or more pharmaceutically acceptable ingredients; ii) an inner coating layer comprising at least one polyol; and iii) an outer flavored coating present on top of the polyol coat and surrounding the core.

The present inventors have found out that the contact between nicotine active and buffering agent potentially jeopardizes the stability of the composition; and by minimizing the same the present invention provides a new and improved product with better stability and release profile.

Further, the buffer in the core ensures that during chewing of the core of the gum product, the suitable buffer agent or substance produces an increase in pH of the saliva which results in an increase in the transmucosal uptake of nicotine in the oral cavity. Also, since the trans-mucosal uptake of nicotine in the oral cavity according to the invention is faster, less nicotine will be swallowed to reach the gastrointestinal (G.I.) tract. The nicotine that reaches the G.I. tract will be subjected to first pass metabolism which reduces the total amount of intact nicotine absorbed.

As used herein, the term "stable" means that the formulation has an acceptable pharmaceutical shelf life of at least 12 months, preferably at least 24 months, when stored at 25 degree C° and at a relative humidity of 60% in a conventional pharmaceutical packaging. Thus in one embodiment of the invention, the chewing gums are chemically and physically stable and have a shelf life of at least 12 months, preferably at least 24 months, when stored at 25 degree C° and at a relative humidity of 60%.

As used herein, the term "nicotine" or "nicotine active" includes pharmaceutically acceptable salts of nicotine, e.g. nicotine bitartrate, nicotine hydrochloride, nicotine dihydrochloride or nicotine sulfate, in particular nicotine bitartrate; and pharmaceutically acceptable nicotine complexes and resins, e.g. nicotine polacrilex.

Typically, the nicotine chewing gums of the present invention comprise the nicotine active in an amount equivalent to 0.1 to 10 mg, preferably 0.5 to 5 mg of nicotine free base.

Not only the amount of the nicotine released from the different parts of the chewing gum product is of value as known in the art, but also, according to the present invention the specific transmucosal uptake from the oral cavity of the nicotine to the systemic circulation of the subject is to a great extent governed by the one or more buffering agents, which account for the provision of a suitable adjustment of the pH of the liquid of the oral cavity.

Suitable buffers are selected from, but are not limited to, alkali metal carbonates, alkali metal bicarbonates, alkaline earth metal carbonates, alkali metal citrates or alkali metal phosphates, or any mixture thereof. Preferably, it is sodium carbonate, potassium carbonate, sodium bicarbonate, potassium bicarbonate, calcium carbonate, potassium citrate or dipotassium phosphate, or any mixture thereof. More preferably, the buffering agent is sodium carbonate, sodium bicarbonate, potassium carbonate, potassium bicarbonate, potassium citrate and dipotassium phosphate, or any mixture thereof. Especially, it is sodium carbonate, sodium bicarbonate, potassium carbonate or potassium bicarbonate, or any mixture thereof.

An embodiment of the buffering agent is characterized by using two different buffering agents in the composition of the chewing gum, e.g. a mixture of an alkali metal bicarbonate with an alkali metal carbonate, such as a mixture of sodium bicarbonate and sodium carbonate.

The dissolution of the one or more buffering agents in the extragranular region of the core provides for optimized adjustment of the pH of the liquid in the oral cavity. Said buffering of the core generates a coated chewing gum product giving improved absorption kinetics of nicotine compared to in the art known chewing gum products.

In order to increase the buffering capacity still further without correspondingly increasing the pH, one may in specific embodiments use a second or auxiliary buffering agent to the first buffering agent, such as e.g., sodium or potassium bicarbonate buffers. The second or auxiliary buffering agent may be selected from the group consisting of alkali metal bicarbonates that are preferred for this purpose. Thus, further embodiments of the invention may comprise a mixture of an alkali metal carbonate or phosphate and alkali metal bicarbonate.

The amount of the buffering agent or agents in the chewing gum composition is preferably sufficient in the specific embodiments to raise the pH of the saliva to above 7, as specified above, to transiently maintain the pH of the saliva in the oral cavity above 7, e.g., pH 7-11.

The present invention provides stable nicotine-containing chewing gum products comprising at least one buffered gum core and one or more coating layers surrounding the core, wherein none of the coating layers is a film-coat.

The present inventors have also found that the coating layers surrounding the gum core are critical to the efficient release of nicotine from the composition and its rapid transmucosal uptake in the oral cavity of the subject. Thus, another feature of the present invention is the judicious selection and application of the various types of coating layers surrounding the gum core, whereby chewing gum compositions free of a film-coat is designed. As a result a superior cost-effective chewing gum product is obtained that provide a high rate of release of nicotine from the chewing gum and an environment in the oral mucosa that enhance buccal absorption of nicotine.

Moreover, the presence of a coating increases the stability of the chewing gum by hampering access of water and oxygen to the uncoated chewing gum core. Typically, polyol coatings come into consideration, of which the sugar free coating formulations are preferred.

Thus, the chewing gums of the invention are coated with a coating that comprises at least one polyol, preferably and surprisingly xylitol. Typically, the amount of coating is approximately 5-50%, preferably 10 -30%, of the uncoated chewing gum. Typically, a sugar free coating mixture comprises a polyol (e.g. maltitol, sorbitol, mannitol, xylitol, erythritol, lactitol or isomalt) a binder, be it, e.g., (a) a gum (e.g. arabic gum, tragacanth gum, guar gum, acacia gum, xanthan gum, alginic acid, salts of alginic acid e.g. sodium alginate, gellan gum, glucomannan gum, carrageenan gum, karaya gum, locust bean gum or tara gum, in particular arabic gum) or (b) a protein-type binder such as gelatin; and optionally flavors, dyes, and traces of any polishing agents used (especially talc and/or Carnauba wax).

In the invention, the chewing gums of the present invention are coated with a coating that comprises a polyol as defined above, in particular xylitol, and a gum as defined above, in particular xanthum gum. Typically, the polyol is present in an amount of 70-95%, especially 80-90%, of the total coating mass. Typically, the gum is present in an amount of 0.05-5%, especially 0.1-1%, of the total coating mass.

The chewing gums of the present invention further comprise of at least one flavored coating. These coatings allow for addition of a large percentage of flavors thereby aiding in prolonging the flavoring sensation.

The flavoring agents may comprise one or more synthetic or natural flavouring or aromatizing agents.

Flavoring agents may be selected from essential oils including distillations, solvent extractions, or cold expressions of chopped flowers, leaves, peel or pulped whole fruit comprising mixtures of alcohols, esters, aldehydes and lactones; essences including either diluted solutions of essential oils, or mixtures of synthetic chemicals blended to match the natural flavour of the fruit, e.g., strawberry, raspberry and black currant; artificial and natural flavours of brews and liquors, e.g., cognac, whisky, rum, gin, sherry, port, and wine; tobacco, coffee, tea, cocoa, and mint; fruit juices including expelled juice from washed, scrubbed fruits such as lemon, orange, and lime; spear mint, pepper mint, wintergreen, cinnamon, cacoe/cocoa, vanilla, liquorice, menthol, eucalyptus, aniseeds nuts (e.g., peanuts, coconuts, hazelnuts, chestnuts, walnuts, colanuts), almonds, raisins; and powder, flour, or vegetable material parts including tobacco plant parts, e.g., genus Nicotiana, in amounts not contributing significantly to the level of nicotine, and ginger.

In a typical embodiment, the chewing gums of the present invention are coated with a flavored coating that further comprises a binder preferably selected from, but not limited to, the group consisting of hydroxypropyl cellulose, hydroxypropylmethyl cellulose, hydroxyethyl cellulose, polyvinyl pyrrolidone, carboxymethyl cellulose, polyvinyl alcohol, sodium alginate, polyethylene glycol, pullulan, tragacanth gum, guar gum, acacia gum, arabic gum, polyacrylic acid, methylmethacrylate copolymer, carboxyvinyl polymer, amylose, high amylose starch, hydroxypropylated high amylose starch, dextrin, pectin, chitin, chitosan, gelatin, zein, gluten, soy protein isolate, whey protein-isolate, casein and mixtures thereof.

In a preferred embodiment of the invention, the chewing gums of the present invention are coated with a flavored coating that comprises a flavoring agent as defined above, in particular mint flavor; and a binder as defined above, in particular hypromellose. Typically, the binder is present in an amount of 0.5-10%, especially 3-5%, of the total coating mass.

The chewing gums of the present composition may further comprise an outer hard coating over the flavored coating whereby the objectionable taste of the API or of other excipient in the flavored coating which directly affects the oral mucosa is avoided.

The chewing gums of the present composition may further comprise an outer glow coating over the flavored coating or outer hard coating.

Further, the coating layers of the chewing gums of the present invention typically do not include any nicotine active and buffering agents.

The present invention provides stable nicotine-containing chewing gum products comprising at least one buffered gum core and one or more coating layers surrounding the core, wherein said buffer in the core is restricted to the extragranular region and wherein none of the coating layers is a film-coat.

In the invention, the chewing gums of the present invention comprise a core comprising gum base, nicotine active, buffering agents and other pharmaceutically acceptable excipients. The said core further comprises a granular and an extragranular region; and the said buffering agents are typically restricted to the extragranular region. The said gum core is further coated with polyol coat, followed by a flavored coat and an outer glow coat.

When the nicotine active and the buffering agent or agents are incorporated in the chewing gum mass in accordance with the present invention, it is possible to employ a wide variety of chewing gum compositions and amounts of the chewing gum base.

The amount of gum base in the chewing gum according to the invention is about 15-85% by weight of the total gum core. The amount of gum base employed for the most desirable slow release of nicotine is usually in the higher ranges when nicotine is employed per se or when an absorbed form is used.

The gum base may be of any conventional nature known in the art. For example it may comprise a gum base of natural or synthetic origin readily available from a commercial source. Natural gum bases include e.g. chicle, jelutong-, lechi de caspi-, soh-, siak-, katiau-, sorwa-, balata-, pendare-, malaya-, and peach gums, natural cautchouc and natural resins such as dammar and mastix. Synthetic gum bases are a mixture of: elastomers (polymers, masticating substances), plasticizer (resin, elastomers, solvent, hydrophobic resin), filler (texturizer, water-insoluble adjuvant), softener (fat), emulsifier, wax, antioxidant, and anti-tacking agents (vinyl polymer, hydrophilic resin). Other examples of gum bases are gums including agar, alginate, arabic gum, carob gum, carrageenan, ghatti gum, guar gum, karaya gum, pectin, tragacanth gum, locust beam gum, gellan gum and xanthan gum.

Other additives may be added optionally to the chewing gum core and/or to coating layers in the chewing gum.

Optional additives comprise at least one or more additive selected from the group consisting of stabilizers, preservatives, e.g., antioxidants; softeners, thickening agents, emulsifiers, glidants, lubricants, sweeteners, gelling agents, enhancers, coloring agents, vitamins, minerals, fluorine and tooth whitening agents and mixtures thereof. According to the invention, at least one of such additives is optionally added to the product.

Examples of gelling agents comprise gum arabic, starch, gelatine, agar, and pectin.

Enhancers are added essentially to improve, i.e., increase, the transmucosal uptake from the oral cavity.

Sweeteners are added essentially to improve the taste.

Sweeteners comprise one or more synthetic or natural sugars, i.e., any form of carbohydrates suitable for use as sweetener, as well as so called artificial sweeteners such as saccarin, sodium saccarin, aspartame, e.g., NutraSweet.RTM., acesulfame K or acesulfame, potassium acesulfame, thaumatin, glycyrrhizin, sucralose, dihydrochalcone, alitame, miraculin, monellin, stevside.

Suitable sweeteners may be selected from the group consisting of sugar alcohols, such as sorbitol, xylitol, single sugars including sugars extracted from sugar cane and sugar beet (sucrose), dextrose (also called glucose), fructose (also called leavulose), and lactose (also called milk sugar); sorbitol, mannitol, glycerol, xylitol, maltitol syrup (or hydrogenated starch hydrolyzate), isomalt, lactitol; and mixtures of sugars including glucose syrup, e.g., starch hydrolysates, containing a mixture of dextrose, maltose and a range of complex sugars, invert sugar syrup, e.g., sucrose inverted by invertase (also called sucrase or sacchrase) containing a mixture of dextrose and fructose, high sugar content syrups such as treacle and honey containing a mixture of particular leavulose, dextrose, maltose, lactitole, sucrose, resins, dextrin and higher sugars; and malt or malt extracts.

Colouring additives may be selected from dyes being approved as a food additive.

Stabilizing additives may be selected from the group consisting of antioxidants including vitamin E, i.e., tocopherole, ascorbic acid, sodium pyrosulfite, butylhydroxytoluene, butylated hydroxyanisole, edetic acid and edetate salts; and preservatives including citric acid, tartaric acid, lactic acid, malic acid, acetic acid, benzoic acid, and sorbic acid. Preferred embodiments comprise an antioxidant as the stabiliser, and even more preferably the antioxidant vitamin E and/or butylated hydroxytoluene (BHT).

Different chewing gum products may be composed depending on the consumers preference and the purpose of use, in respect of the nicotine level, nicotine distribution and other additives.

The present invention further provides nicotine chewing gums that can be manufactured by tabletting techniques, and thus, do not require the specific (e.g. melt extrusion) equipment necessary in the manufacture of conventional chewing gums.

The term "tabletting" on the one hand includes, preferably, conventional tabletting techniques but also, on the other hand, any sophisticated ones. They all have in common the application of force, i.e. the compression of the mixture of all components of the chewing gum.

Coated chewing gum products according to the invention can be maintained in several production steps depending on the total number of cores and the total number of coated layers to be included.

A typical method for the production of the coated chewing gum according to the invention is disclosed. The method comprises the steps of
a) providing a nicotine containing chewing gum core that is buffered,
b) providing at least one coating layer, and
c) coating the buffered chewing gum core with the at least one coating layer.

In a typical embodiment of the invention, the core composition may be formed simply by mixing, rolling and scoring or compression of the chewing gum base with the nicotine-active, e.g., the nicotine-ion exchanger complex, or the nicotine as a free base or a salt. Before adding any solid component, except for the gum base, it is desirable to grind and size the solid component first, to ensure good distribution.

In the present invention, the nicotine containing chewing gum was prepared by the following steps:
a) The nicotine active is dispersed in liquid flavoring agent;
b) The dispersion obtained in step a) is then adsorbed on gum base;
c) The nicotine active adsorbed gum base obtained step b) are then blended with other pharmaceutically acceptable excipients to get a uniform free-flowing mass;
d) The blend obtained in step c) is then granulated and compressed to form tablets.

Chewing gums produced by compression, i.e., tabletted gums are made out of a special gum base.

Such a gum base gives a rapid hydration of the cud in the mouth. High velocity mixers can be used for granulation to give correctly sized particles of the mixture. This mixture is then compressed in a tablet machine.

According to the method disclosed in the invention the coating of the buffered chewing gum core with at least one layer of the at least one coating comprises the steps of
a) press coating, and/or
b) polyol coating, and/or
c) spray coating, and/or
c) melt coating.

The present invention, as described herein, provides an advantageous method of dosing active ingredients into a large amount of chewing gum composition, which facilitates an exact dosing of the active ingredients in a series of tablets. This means that large-scale production of chewing gum tablets comprising pharmaceutically active ingredients is made possible with improved results concerning distribution of the active ingredients in the tablets and thereby an improved uniformity in content of active ingredient in a series of chewing gum tablets.

The product may then be analyzed and further wrapped. The analysis of nicotine uptake and effect according to the invention may be done according to standard procedure known in the art, e.g., using a bioanalysis for the determination of nicotine or its metabolites in the plasma of a subject.

The present invention provides efficient and effective chewing gum compositions for use in nicotine replacement therapy, which provide rapid nicotine release in the oral cavity and fast--but non-toxic, pharmaceutically acceptable buccal absorption of nicotine, and which is characterized by comprising a compressible chewing gum base that allows the chewing gum to be manufactured by tableting at least one nicotine active and at least one buffering agent.

Fast--but non-toxic, pharmaceutically acceptable--buccal absorption of nicotine typically is further characterized by high in-vivo truly delivered total doses of nicotine in the mouth of a patient over a time period of 30 minutes, e.g. by a delivery of at least 80%--preferably at least 90% and more preferably at least 95%--of the original amount of nicotine in the gum over 30 min.

Preferably, rapid nicotine release is characterized by an in vitro release of at least 60% in 5 minutes (250 chews), of at least 75% (more preferably at least 85%) in 10 minutes (500 chews) and more than 90% in 30 minutes (1500 chews) of the nicotine assay value. The principles, preferred embodiments, and modes of operation of the present invention have been described in the foregoing specification.

The following examples further illustrate the invention.

### Examples:

### Example: Chewing gum composition of nicotine polacrilex

### I) Preparation of buffered gum core:

| **Component** | **% w/w** |
|---|---|
| **Nicotine polacrilex 20%** | 0.69 |
| **Gum Base** | 76.0 |
| **Sodium carbonate anhydrous** | 1.02 |
| **Sodium hydrogen carbonate** | 0.51 |
| **Silica, colloidal hydrated** | 1.67 |
| **Magnesium stearate** | 1.67 |
| **Mint flavor** | 0.83 |
| **Acesulfame potassium** | 0.45 |

### Procedure:

a) Nicotine polacrilex is dispersed in the mint flavor manually, and adsorbed to a part of the gum base,
b) Colloidal silica is added to the mixer in step a) and kneaded for sufficient time at suitable speed to form granules,
c) The remaining ingredients, except magnesium stearate, are mixed with the remaining part of the gum base,
d) The extragranular mass obtained in step c) is then blended with the API adsorbed granules of step (b),
e) The blend obtained in step d) is then lubricated and compressed.

### II) Preparation of Sub coat:

| **Component** | **% w/w** |
|---|---|
| **Xylitol** | 10.5 |
| **Xanthum gum** | 0.06 |
| **Talc** | 0.20 |
| **dye** | 0.30 |
| **Purified water** | -- |

### Procedure:

f) Xanthum gum is dissolved in required quantity of purified water under continuous stirring to get a clear viscous solution ,
g) Xylitol, talc and the dye are added to the solution of step f) and stirred continuously.

### III) Preparation of flavored coat:

| **Component** | **% w/w** |
|---|---|
| **Mint flavor** | 0.75 |
| **Xylitol** | 2.75 |
| **Hypromellose** | 0.67 |
| **Talc** | 0.03 |
| **Dye** | 0.1 |
| **Purified water** | -- |

### Procedure:

h) Hypromellose is dissolved in required quantity of purified water under continuous stirring to get a clear viscous solution ,
i) The remaining ingredients are added to the solution of step h) one at a time and stirred continuously.

### IV) Preparation of coated chewing gum:

j) The API containing gum cores obtained in step e) are transferred to a coating pan and the coating dispersion obtained in step g) was sprayed on to the cores, and the coated cores were dried.
k) The coating dispersion obtained in step i) was sprayed on to the polyol coated gum cores obtained in step j).
l) The coated gum cores of step k) were then coated with a solution Insta glow in purified water.

## Claims

1. A stable chewing gum for use in nicotine replacement therapy comprising:
i) a core having a) a granular region comprising a compressible chewing gum base that is suitable for tabletting at ambient temperature, at least one nicotine active, and optionally, one or more pharmaceutically acceptable ingredients; and b) an extragranular region comprising buffering agents and optionally, one or more pharmaceutically acceptable ingredients;
ii) an inner coating layer comprising at least one polyol; and
iii) an outer flavored coating present on top of the polyol coat and surrounding the core.

2. A chewing gum for use according to claim 1, which is **characterized by** comprising the at least one nicotine active in an amount corresponding to from 0.1 to 10 mg of nicotine free base.

3. A chewing gum for use according to claim 1, wherein the nicotine active is selected from the group consisting of pharmaceutically acceptable salts of nicotine and pharmaceutically acceptable, cellulose-free, nicotine complexes and resins.

4. A chewing gum for use according to claim 1, wherein the nicotine active is nicotine polacrilex.

5. A chewing gum for use according to claim 1, wherein the buffering agent is selected from the group consisting of alkali metal carbonates, alkali metal bicarbonates, alkaline earth metal carbonates, alkali metal citrates or alkali metal phosphates, or any mixture thereof.

6. A chewing gum for use according to claim 1, wherein the coating comprises a polyol selected from the group consisting of maltitol, sorbitol, mannitol, xylitol, erythritol, lactitol, isomalt or any mixture thereof.

7. A chewing gum for use according to claim 1, wherein the chewing gum composition further comprise at least one or more additives selected from the group consisting of stabilizers, preservatives, e.g., antioxidants; softeners, thickening agents, emulsifiers, glidants, lubricants, sweeteners, gelling agents, enhancers, coloring agents, vitamins, minerals, fluorine and tooth whitening agents and mixtures thereof.

8. A method for preparation of a stable chewing gum for use according to any one of the preceding claims for use in nicotine replacement therapy, wherein the method comprises of the steps of:
i. The nicotine active is dispersed in liquid flavoring agent;
ii. The dispersion obtained in step i) is then adsorbed on gum base;
iii. The nicotine active adsorbed gum cores obtained step ii) are then blended with other pharmaceutically acceptable excipients to get a uniform free-flowing mass;
iv. The blend obtained in step iii) is then granulated and compressed to form tablets.

## Patentansprüche

1. Stabiles Kaugummi zur Verwendung bei der Nicotinersatztherapie, umfassend:
i) einen Kern mit a) einem granulären Bereich, der eine zum Tablettieren bei Umgebungstemperatur geeignete komprimierbare Kaugummibasis, mindestens einen Nicotinwirkstoff und gegebenenfalls einen oder mehrere pharmazeutisch unbedenkliche Bestandteile umfasst; und b) einem extragranulären Bereich, der Puffermittel und gegebenenfalls einen oder mehrere pharmazeutisch unbedenkliche Bestandteile umfasst;
ii) eine mindestens ein Polyol umfassende innere Überzugsschicht; und
iii) eine äußere aromatisierte Beschichtung, die auf der Polyolschicht liegt und den Kern umgibt.

2. Kaugummi zur Verwendung nach Anspruch 1, **dadurch gekennzeichnet, dass** es den mindestens einen Nicotinwirkstoff in einer Menge enthält, die 0,1 bis 10 mg freier Nicotinbase entspricht.

3. Kaugummi zur Verwendung nach Anspruch 1, wobei der Nicotinwirkstoff aus der aus pharmazeutisch unbedenklichen Salzen von Nicotin und pharmazeutisch unbedenklichen cellulosefreien Nicotinkomplexen und -harzen bestehenden Gruppe ausgewählt ist.

4. Kaugummi zur Verwendung nach Anspruch 1, wobei es sich bei dem Nicotinwirkstoff um Nicotinpolacrilex handelt.

5. Kaugummi zur Verwendung nach Anspruch 1, wobei das Puffermittel aus der aus Alkalicarbonaten, Alkalihydrogencarbonaten, Erdalkalicarbonaten, Alkalicitraten oder Alkaliphosphaten oder einer Mischung davon bestehenden Gruppe ausgewählt ist.

6. Kaugummi zur Verwendung nach Anspruch 1, wobei die Beschichtung ein aus der aus Maltit, Sorbit, Mannit, Xylit, Erythrit, Lactit, Isomalt oder einer beliebigen Mischung davon bestehenden Gruppe ausgewähltes Polyol umfasst.

7. Kaugummi zur Verwendung nach Anspruch 1, wobei die Kaugummizusammensetzung weiterhin mindestens einen oder mehrere aus der aus Stabilisatoren, Konservierungsstoffen, z. B. Antioxidationsmitteln, Weichmachern, Verdickungsmitteln, Emulgatoren, Gleitmitteln, Schmiermitteln, Süßstoffen, Geliermitteln, Verstärkern, Farbstoffen, Vitaminen, Mineralien, Fluor und Zahnaufhellungsmitteln und Mischungen davon bestehenden Gruppe ausgewählte Zusatzstoffe umfasst.

8. Verfahren zur Herstellung eines stabilen Kaugummis zur Verwendung nach einem der vorhergehenden Ansprüche zur Verwendung in der Nicotinersatztherapie, wobei das Verfahren die folgenden Schritte umfasst:
i. der Nicotinwirkstoff wird in einem flüssigen Aromastoff dispergiert;
ii. die in Schritt i) erhaltene Dispersion wird dann auf der Kaugummibasis adsorbiert;
iii. die in Schritt ii) erhaltenen Kaugummikerne mit adsorbiertem Nicotinwirkstoff werden dann unter Erhalt einer einheitlichen, frei fließenden Masse mit anderen pharmazeutisch unbedenklichen Exzipienten gemischt;
iv. die in Schritt iii) erhaltene Mischung wird dann granuliert und zu Tabletten verpresst.

## Revendications

1. Gomme à mâcher stable pour utilisation dans une thérapie de remplacement de la nicotine comprenant :
i) un noyau comportant a) une région granulaire comprenant une base de gomme à mâcher compressible qui est adaptée pour la fabrication de comprimés à température ambiante, au moins un composant actif nicotinique et, facultativement, un ou plusieurs composants pharmaceutiquement acceptables ; et b) une région extragranulaire comprenant des agents tampons et, facultativement, un ou plusieurs composants pharmaceutiquement acceptables ;
ii) une couche d'enrobage interne comprenant au moins un polyol ; et
iii) un enrobage aromatisé externe présent au-dessus de la couche de polyol et entourant le noyau.

2. Gomme à mâcher pour utilisation selon la revendication 1, **caractérisée en ce qu'**elle comprend l'au moins un composant actif nicotinique en une quantité correspondant à 0,1 à 10 mg de nicotine base libre.

3. Gomme à mâcher pour utilisation selon la revendication 1, dans laquelle le composant actif nicotinique est choisi dans le groupe constitué de sels de nicotine pharmaceutiquement acceptables et de complexes et résines de nicotine pharmaceutiquement acceptables, sans cellulose.

4. Gomme à mâcher pour utilisation selon la revendication 1, dans laquelle le composant actif nicotinique est la nicotine polacrilex.

5. Gomme à mâcher pour utilisation selon la revendication 1, dans laquelle l'agent tampon est choisi dans le groupe constitué de carbonates de métal alcalin, des bicarbonates de métal alcalin, des carbonates de métal alcalino-terreux, des citrates de métal alcalin ou des phosphates de métal alcalin, ou un mélange quelconque de ceux-ci.

6. Gomme à mâcher pour utilisation selon la revendication 1, dans laquelle l'enrobage comprend un polyol choisi dans le groupe constitué du maltitol, du sorbitol, du mannitol, du xylitol, de l'érythritol, du lactitol, de l'isomalt ou un mélange quelconque de ceux-ci.

7. Gomme à mâcher pour utilisation selon la revendication 1, dans laquelle la composition de gomme à mâcher comprend en outre au moins un ou plusieurs additifs choisis dans le groupe constitué de stabilisants, conservateurs, par exemple des antioxydants ; adoucissants, agents épaississants, émulsifiants, agents glissants, lubrifiants, édulcorants, agents gélifiants, exhausteurs de goût, agents colorants, vitamines, minéraux, fluor et agents de blanchiment des dents et des mélanges de ceux-ci.

8. Procédé de préparation d'une gomme à mâcher stable pour utilisation selon l'une quelconque des revendications précédentes pour une utilisation dans une thérapie de remplacement de la nicotine, le procédé comprenant les étapes suivantes :
i. le composant actif nicotinique est dispersé dans un agent aromatisant liquide ;
ii. la dispersion obtenue à l'étape i) est ensuite adsorbée sur une base de gomme ;
iii. les noyaux de gomme sur laquelle le composant actif nicotinique est adsorbé obtenus à l'étape ii) sont ensuite mélangés avec d'autres excipients pharmaceutiquement acceptables pour obtenir une masse uniforme et fluide ;
iv. le mélange obtenu à l'étape iii) est ensuite granulé et comprimé pour former des comprimés.
